(19) **Europäisches Patentamt / European Patent Office / Office européen des brevets**

(11) **EP 4 740 888 A1**

(12) **EUROPEAN PATENT APPLICATION**
published in accordance with Art. 153(4) EPC

(43) Date of publication:
**13.05.2026 Bulletin 2026/20**

(21) Application number: **24838484.4**

(22) Date of filing: **03.06.2024**

(51) International Patent Classification (IPC):
**A61B 34/30** (2016.01)

(52) Cooperative Patent Classification (CPC):
**A61B 34/00; A61B 34/20; A61B 34/30;
A61B 34/35; A61B 34/37; B25J 9/16; G06V 40/20**

(86) International application number:
**PCT/CN2024/096927**

(87) International publication number:
**WO 2025/011226 (16.01.2025 Gazette 2025/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **07.07.2023 CN 202310831557
28.07.2023 CN 202310939859**

(71) Applicant: **Beijing Surgerii Robotics Company
Limited
Beijing 100192 (CN)**

(72) Inventors:
• **XU, Kai**
**Beijing 100192 (CN)**
• **ZHAO, Jiangran**
**Beijing 100192 (CN)**
• **SHI, Hao**
**Beijing 100192 (CN)**
• **HU, Yonghui**
**Beijing 100192 (CN)**

(74) Representative: **karo IP
Patentanwälte PartG mbB
Steinstraße 16-18
40212 Düsseldorf (DE)**

(54) **MOTION CONTROL METHOD FOR ROBOT SYSTEM, AND SURGICAL ROBOT**

(57) The present disclosure relates to the field of medical instruments, and discloses a motion control method of robot system and surgical robot. The robot system comprises a mobile platform, at least one motion arm disposed on the mobile platform, and an input device for controlling the motion of the motion arm and the mobile platform. The control method includes receiving input information corresponding to user input from an input device, and controlling the motion arm or the mobile platform to move based on the input information. Based on the user's operational input to the input device, the motion of the motion arm or the mobile platform may be easily achieved, making the preoperative positioning operation convenient and easy, and reducing preoperative preparation time.

FIG. 2

**Description**

**Cross-reference to Relevant Applications**

The present application claims the priority of a Chinese Patent Application with Application No. 2023109398598, filed on July 28, 2023, entitled "Motion control method of robot system and surgical robot", and Application No. 2023108315579, filed on July 7, 2023, entitled "Motion control method of robot system and surgical robot", the full disclosure of these applications is incorporated herein by reference in their entirety.

**Technical Field**

The present disclosure relates to the field of medical instruments, in particular to a motion control method of robot system and surgical robot.

**Background**

Compared with traditional open surgery, minimally invasive endoscopic surgery has the advantages of less surgical trauma, faster postoperative recovery, lower postoperative infections and complications, and has been widely used. Regarding the existing endoscopic surgical robot system, surgical instruments are carried by a motion arm. According to different patients and surgical procedures, it is necessary to adjust the positioning of the motion arm before or during the surgery so that the surgical instrument can be adjusted to the designated position for the surgery. During the surgery, the surgeon in charge controls the surgical effectors at the distal end terminal of the surgical instrument to realize surgeries at different parts by using remote operation mode.

At present, using surgical robots to achieve surgical procedures mainly includes three processes: preoperative positioning, surgical preparation, and postoperative organization. In the preoperative positioning and preparation process, the surgical assistant (such as a nurse or doctor) needs to move the surgical robot to the patient's side, and then adjust at least one motion arm to the appropriate position and orientation according to the type of surgery and the surgical position, so that the at least one motion arm can be connected with the trocar, so that the surgical instrument on the motion arm can enter the corresponding position in the human body requiring surgery through the trocar. During the surgery, the surgical instrument is controlled by remote operation and driven by the instructions from the input end to reach various positions and angles within the range, so as to achieve various operating actions.

The movement of existing surgical robot systems relies on manual dragging, which is inconvenient and time-consuming to operate; the pose of the motion arm is usually automatically adjusted by the system, and adjustments for some specific poses are time-consuming. The convenience of movement of the surgical robot system, as well as the abilities for pose adjusting and positioning of the external motion arm in the space, directly affect the preoperative preparation time and operational experience.

**Summary of the Invention**

In some embodiments, the present disclosure provides a motion control method for a robot system, the robot system comprises a mobile platform, at least one motion arm disposed on the mobile platform, and an input device, the input device is used to control a motion of the motion arm and the mobile platform, the control method comprises:

receiving, from the input device, input information corresponding to user input; and
controlling, based on the input information, the motion arm or the mobile platform to move.

In some embodiments, the present disclosure further provides a surgical robot comprises:

a mobile platform;
a motion arm, the motion arm is movably disposed on the mobile platform;
a grip disposed on the motion arm and used to control motions of the motion arm and the mobile platform, the grip comprises at least one force sensor, the force sensor is used to detect a force or torque externally applied to the grip; and
a controller configured to execute the control method as described in any embodiment of the present disclosure.

In some embodiments, the present disclosure further provides a computer storage medium, the storage medium comprising at least one instruction, the at least one instruction is executed by a processor to configure the processor to execute the control method as described in any embodiment of the present disclosure.

In some embodiments, the present disclosure further provides a computer system comprises:

a storage medium comprising at least one instruction; and
a processor configured to execute the at least one instruction to configure the processor to execute the control method as described in any embodiment of the present disclosure.

## Brief Description of the Drawings

In order to explain the technical solutions in the embodiments of the present disclosure more clearly, the accompanying drawings used in the description of the embodiments of the present disclosure will be briefly introduced below. The accompanying drawings in the following description only show some of the embodiments of the present disclosure, and for those of ordinary skill in the art, other embodiments would also have been obtained from the contents of the embodiments of the present disclosure and these accompanying drawings without involving any inventive effort.

FIG. 1 illustrates a flowchart of a motion control method of a robot system according to some embodiments of the present disclosure;
FIG. 2 illustrates a schematic block diagram of a robot system according to some embodiments of the present disclosure;
FIG. 3 illustrates a structural diagram of a motion arm of a robot system according to some embodiments of the present disclosure;
FIG. 4 illustrates a structural schematic diagram of a master manipulator according to some embodiments of the present disclosure;
FIG. 5 illustrates a coordinate diagram of a motion arm of a robot system according to some embodiments of the present disclosure;
FIG. 6 illustrates a flowchart of a motion control method of a motion arm of a robot system according to some embodiments of the present disclosure;
FIG. 7 illustrates a schematic block diagram of a robot system according to other embodiments of the present disclosure;
FIG. 8 illustrates a flowchart of a motion control method of a motion arm of a robot system according to other embodiments of the present disclosure;
FIG. 9 illustrates a flowchart of a motion control method of a mobile platform of a robot system according to some embodiments of the present disclosure;
FIG. 10 illustrates a simplified schematic diagram of a control point of a mobile platform of a robot system according to some embodiments of the present disclosure;
FIG. 11 illustrates a schematic diagram of a control point coordinate of a mobile platform of a robot system according to some embodiments of the present disclosure;
FIG. 12 illustrates a structural schematic diagram of a surgical robot according to some embodiments of the present disclosure.

## Detailed Description

To make the solved technical problems, used technical solutions, and achieved technical effects of the present disclosure more clearly, the technical solutions of the embodiments of the present disclosure will be further described in detail below with reference to the accompanying drawings. Obviously, the described embodiments are only exemplary embodiments, but not all of embodiments, of the present disclosure.
In the description of the present disclosure, it should be noted that, orientational or positional relationships indicated by the terms "center", "upper", "lower", "left", "right", "vertical", "horizontal", "inner", "outer" and the like are the orientational or positional relationships shown based on the accompanying drawings, and are only for ease of describing the present disclosure and simplifying the description, rather than indicating or implying that the apparatus or element referred to must have a specific orientation or be constructed and operated in a specific orientation, and therefore cannot be construed as limiting the present disclosure. In addition, the terms "first" and "second" are used for descriptive purposes only, and cannot be understood as indicating or implying relative importance. In the description of the present disclosure, it should be noted that, unless otherwise specified and defined, the term "mount", "connected", and "connect", or "couple" should be comprehended in a broad sense. For example, the term may be a fixed connection or a detachable connection; or may be a mechanical connection or an electrical connection; may be a direct connection or an indirect connection via an intermediate medium; or may be internal communication between two elements. For those of ordinary skill in the art, specific meanings of the foregoing terms in the present disclosure may be understood based on specific situations. In the present disclosure, an end close to an operator (e.g., a surgeon) is defined as a proximal end, a proximal portion, a rear end, or a rear portion, and an end close to a patient who requires surgery is defined as a distal end, a distal portion, a front end, or a front portion. It may be understood by those skilled in the art that the embodiments of the present disclosure may be used for a medical instrument or a surgical robot, and may also be used for other non-medical apparatus.

In the present disclosure, the term "position" refers to a positioning of an object or a portion of the object in three-dimensional space (e.g., variations in Cartesian X, Y, and Z coordinates may be used to describe three translational degrees of freedom, e.g., three translational degrees of freedom along the Cartesian X-axis, Y-axis, and Z-axis respectively). In the present disclosure, the term "orientation" refers to a rotation setting of an object or a portion of the object (e.g., three rotational degrees of freedom, which may be described using roll, pitch, and yaw). In the present disclosure, the term "pose" refers to a combination of a position and an orientation of an object or a portion of the object. For example, it may be described using six parameters in the six degrees of freedom mentioned above. In the present disclosure, the configuration or pose of a motion arm or a portion thereof can be represented by a set of joint values of the joints of the motion arm (such as a one-dimensional matrix composed of these joint values). In the present disclosure, the joint values of the joint may include an angle of a respective joint rotating relative to a corresponding joint axis or a distance of the respective joint moving relative to an initial position. In the present disclosure, a reference coordinate system may be understood as a coordinate system capable of describing a pose of an object. According to actual positioning requirements, the reference coordinate system may be chosen to take an origin of a virtual reference object or an origin of a real reference object as an origin of the coordinate system. In the present disclosure, the pose of the motion arm or a portion (for example, an end) thereof may refer to a pose of the coordinate system defined by the motion arm or the portion thereof relative to the reference coordinate system. In the present disclosure, unless otherwise specified, the position and orientation of the motion arm refer to the position and orientation of the motion arm control point coordinate system, and the position and orientation of the mobile platform refer to the position and orientation of the mobile platform coordinate system.

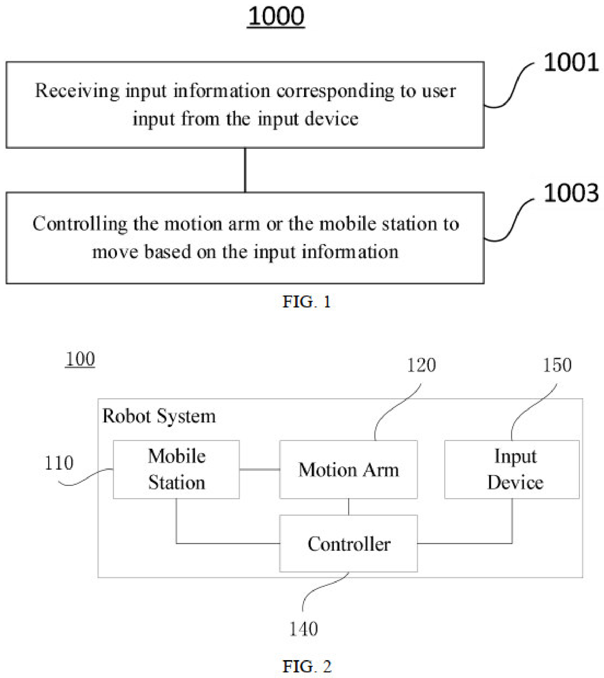

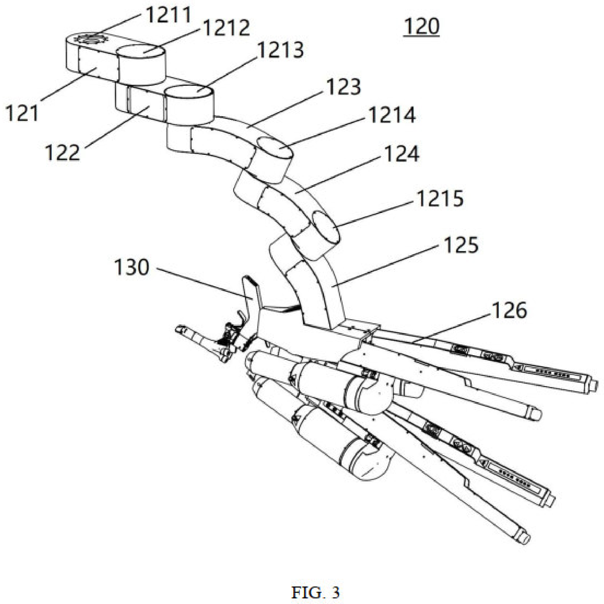

FIG. 1 illustrates a flowchart of a motion control method 1000 of a robot system 100 according to some embodiments of the present disclosure; FIG. 2 illustrates a schematic block diagram of a robot system 100 according to some embodiments of the present disclosure; FIG. 3 illustrates a structural diagram of a motion arm 120 of a robot system 100 according to some embodiments of the present disclosure. The method 1000 may be implemented or performed by hardware, software or firmware. In some embodiments, the method 1000 may be performed by a robot system (e.g., a controller 140 of the robot system 100 as shown in FIG. 2). In some embodiments, the method 1000 may be implemented as computer-readable instructions. These instructions may be read and executed by a general-purpose processor or a dedicated processor. For example, the processor of the controller 140 of the robot system 100 is configured to execute the method 1000. In some embodiments, these instructions may be stored on a computer-readable medium.

In some embodiments, as shown in FIG. 2, the robot system 100 includes a mobile platform 110, at least one motion arm 120 disposed on the mobile platform 110, and an input device 150. The input device 150 may be communicatively connected with the controller 140, and the controller 140 can be communicatively connected with the mobile platform 110 and the motion arm 120, for example, via cable connections, or via wireless connections, to achieve communication among the input device 150, the controller 140, the motion arm 120 and the mobile platform 110. The input device 150 may be used to control the motion of the motion arm 120 and the mobile platform 110. Based on the user's operational input to the input device, the motion of the motion arm or the mobile platform may be easily achieved, making the preoperative positioning operation convenient and easy, and reducing preoperative preparation time.

It should be understood that the robot system 100 may include a motion arm 120. The motion arm 120 may include one or more arm bodies, one or more joints connecting the one or more arm bodies, and one or more joint motors driving the one or more joints. As shown in FIG. 3, the motion arm 120 may include a plurality of arm bodies (such as arm bodies 121-125) and a plurality of joints (such as joints 1211-1215) connecting the plurality of arm bodies, and a plurality of joint motors (not shown in the figure) may be disposed at the plurality of joints. The motions of the plurality of joints are driven through the plurality of joint motors to drive the motions of the plurality of arm bodies. Specifically, as shown in FIG. 3, the motion arm 120 may include a first transverse arm 121, a second transverse arm 122, a first arc arm 123, a second arc arm 124, and a third arc arm 125. The motion arm 120 may also include a first transverse arm rotation joint 1211, a second transverse arm rotation joint 1212, a first arc arm rotation joint 1213, a second arc arm rotation joint 1214, and a third arc arm rotation joint 1215. The above are only examples. It should be understood that the number of arm bodies or joints of the motion arm 120 may also be other quantities; or the motion arm 120 may also include a vertical-arm and the corresponding motion joints thereof; or the motion arm 120 does not include an arc-shaped arm and the corresponding rotation joints thereof, but includes one or more arm bodies and the corresponding motion joints thereof that can achieve similar functions.

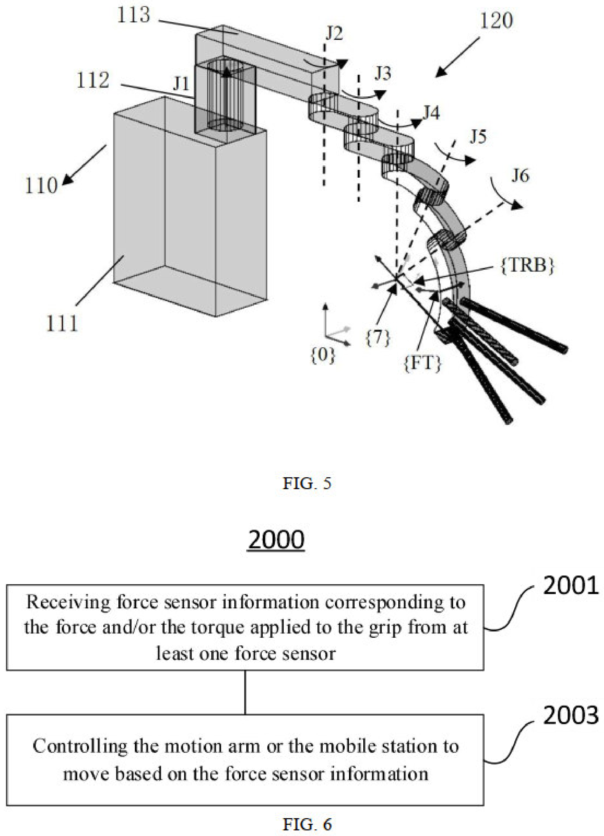

In some embodiments, the mobile platform 110 may be a patient-side cart. As shown in FIG. 5, the mobile platform 110 may include a base 111, a column 112 disposed on the base 111, a lifting joint J1 connected to the column 112, a column lifting motor driving the column lifting joint J1, and a crossbeam 113 disposed on the column 112. The base 111 includes one or more active casters (not shown in the figure) and one or more active caster motors that drive the active casters. The active caster motor may drive the motion of the active caster, thereby achieving forward, backward, or turning motion of the mobile platform 110. The column lifting motor can drive the column 112 to ascend and descend, thereby achieving the ascending and descending of the crossbeam 113 of the mobile platform 110. The above is only an example, and it should be understood that the mobile platform 110 may also include horizontal movement joints and/or rotation joints. For example, the crossbeam 113 may be connected to the column 112 through a horizontal movement joint, or the crossbeam 113 may be connected to the motion arm 120 through a rotation joint.

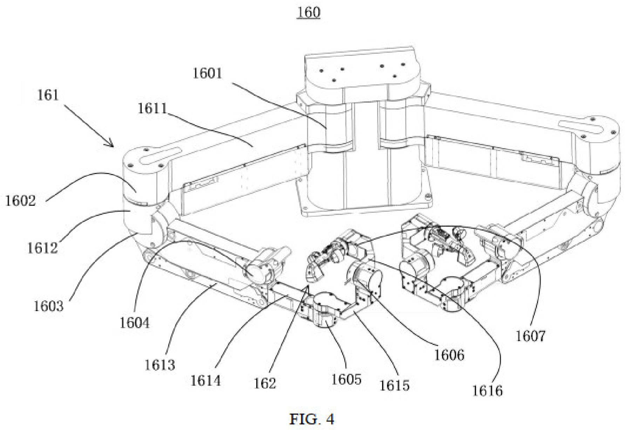

In some embodiments, the input device 150 may include at least one of the following: a grip 130 disposed on the motion arm 120, an operating handle (e.g., the master manipulator 160 shown in FIG. 4), an input interface, or at least one mechanical button. In some embodiments, the input device 150 may include a motion arm adjustment mode selection key or a mobile platform adjustment mode selection key. It should be understood that the motion arm adjustment mode selection key may include a motion arm position adjustment mode selection key and a motion arm orientation adjustment mode selection key. For example, the robot system 100 may receive the selection of the adjustment mode selection key for the motion arm 120 or the adjustment mode selection key for the mobile platform 110 from the operator through the input device 150, so as to enter the adjustment mode of the motion arm 120 (for example, the pose adjustment mode, the position adjustment mode or the orientation adjustment mode), or enter the adjustment mode of the mobile platform 110.

As shown in FIG. 1, in step 1001, the input information corresponding to the user input is received from the input device. In some embodiments, the input device 150 may include an operating handle, the user input includes operating actions to the operating handle, and the input information includes handle operating information. It should be understood that the operating information of the handle corresponds to the operating action to the operating handle. In some embodiments, the operating handle is configured to move in at least two dimensions. It should be understood that the operating handle can include various suitable structures, such as a joystick, a handle with a robotic arm, a wireless handle, and so on. The operating actions to the operating handle may include but not limited to: moving the operating handle left and right, moving the operating handle up and down, moving the operating handle forward and backward, deflecting the operating handle left and right, leaning the operating handle up and down, rolling the operating handle left and right, etc. For example, the robot system 100 may receive a selection of the adjustment mode selection key for the motion arm 120 from the operator through the input device 150, so as to enter the adjustment mode of the motion arm 120 (such as pose adjustment mode, position adjustment mode, or orientation adjustment mode). In the adjustment mode for the motion arm 120, the motion arm 120 may be controlled to move left and right by moving the operating handle left and right; the motion arm 120 may be controlled to deflect left and right by deflecting the operating handle left and right; the motion arm 120 may be controlled to move up and down by moving the operating handle up and down; the motion arm 120 may be controlled to lean up and down by leaning the operating handle up and down; etc. It should be understood that the motion center of the motion arm 120 may correspond to the control point of the motion arm.

For example, the robot system 100 may receive a selection of the adjustment mode selection key for the mobile platform 110 from the operator through the input device 150, so as to enter the adjustment mode of the mobile platform 110. In the adjustment mode of the mobile platform 110, the mobile platform 110 may be controlled to move forward and backward by moving the operating handle forward and backward; the mobile platform 110 may be controlled to turn left and right by turning the operating handle left and right, and the mobile platform 110 may be controlled to ascend and descend by moving the operating handle up and down. The motion center of the mobile platform 110 may correspond to the control point of the mobile platform. It should be understood that the input device 150 may also be a joystick, the motion of the motion arm 120 or the mobile platform 110 may be controlled by operating the joystick.

In some embodiments, the input device 150 may include an input interface, the user input includes operating actions to the input interface, and the input information includes interface operating information. It should be understood that the interface operating information corresponds to the operating actions to the input interface. In some embodiments, the input interface includes input keys configured to adjust in at least two dimensions. It should be understood that the input interface may include any suitable implementation form, such as touch-based interactive interfaces, graphical interfaces that can be controlled by tools such as mice or keyboards, and so on. The operating actions to the input interface may include selecting inputs for multiple input keys. For example, input keys may include but are not limited to: moving the input key left and right; moving the input key up and down; moving the input key forward and backward; deflecting the input key left and right; leaning the input key up and down; rolling the input key left and right, etc. For example, In the adjustment mode for the motion arm 120, the motion arm 120 can be controlled to move left and right by moving the input key left and right; the motion arm 120 can be controlled to deflect left and right by deflecting the input key left and right; the motion arm 120 can be controlled to move up and down by moving the input key up and down; the motion arm 120 can be controlled to lean up and down by leaning the input key up and down; etc. The input forms may include clicking, sliding, dragging, and so on. In the adjustment mode of the mobile platform 110, the mobile platform 110 may be controlled to move forward and backward by moving the input key forward and backward; the mobile platform 110 may be controlled to turn left and right by turning the input key left and right, and the mobile platform 110 may be controlled to ascend and descend by moving the input key up and down.

In some embodiments, the operating actions to the input interface may include image adjustments to 3D images (such as 3D images of mobile platform 110 or motion arm 120). For example, users may perform operations such as left and right movement, up and down movement, forward and backward movement, left and right deflection, up and down lean, and left and right roll on the 3D image on the interface through touch control to control the motion of the mobile platform 110 or the motion arm 120. It should be understood that the motion center of the 3D image may coincide with the image of the control center of the motion arm 120 or the motion center of the mobile platform 110. The input interface may also include adjustment instructions for 3D images, such as left and right movement arrows, up and down adjustment arrows, forward and backward movement arrows, yaw adjustment arrows, roll adjustment arrows, pitch adjustment arrows, and so on. By operating (such as clicking, sliding, or dragging) the arrows, input for motion control of the mobile platform 110 or the motion arm 120 can be achieved.

In some embodiments, the input device 150 may include mechanical buttons, the user input includes operating actions to the mechanical buttons, and the input information includes button operating information. It should be understood that button operating information corresponds to the operating actions to the mechanical buttons respectively. In some embodiments, at least one mechanical button includes multiple buttons configured to adjust in at least two dimensions. It should be understood that the operating actions to the mechanical buttons may include selecting inputs for multiple buttons. For example, buttons may include but are not limited to: mode adjustment buttons, left and right movement buttons, up and down movement buttons, forward and backward movement buttons, left and right deflection buttons, up and down lean buttons, left and right roll buttons, etc. For example, In the adjustment mode for the motion arm 120, the motion arm 120 can be controlled to move left and right by moving the button left and right; the motion arm 120 can be controlled to deflect left and right by deflecting the button left and right; the motion arm 120 can be controlled to move up and down by moving the button up and down; the motion arm 120 can be controlled to lean up and down by leaning the button up and down; etc. In the adjustment mode of the mobile platform 110, the mobile platform 110 may be controlled to move forward and backward by moving the button forward and backward; the mobile platform 110 may be controlled to turn left and right by turning the button left and right, and the mobile platform 110 may be controlled to ascend and descend by moving the button up and down.

As shown in FIG. 1, in step 1003, the motion arm or mobile platform is controlled to move based on the input information. In some embodiments, the input device 150 is an operating handle, and the input information may include handle operating information. The controller 140 of the robot system 100 may receive handle operating information corresponding to the operating actions to the operating handle from the operating handle. Based on the handle operating information, the movement of the motion arm 120 or the mobile platform 110 is controlled. In some embodiments, the input device 150 is an input interface, and the input information includes interface operating information. The controller 140 of the robot system 100 may receive interface operating information corresponding to the operating actions to the input interface from the input interface. Based on the interface operating information, the movement of the motion arm 120 or the mobile platform 110 is controlled. In some embodiments, the input device is a mechanical button, and the input information includes button operating information. The controller 140 of the robot system 100 may receive button operating information corresponding to the operating actions to the mechanical button from the mechanical button. Based on the button operating information, the movement of the motion arm 120 or the mobile platform 110 is controlled.

In some embodiments, the motion of mobile platform 110 may include at least one of the following: forward and backward movement of the mobile platform, left and right turning of the mobile platform, ascent and descent of the mobile platform, and so on. In some embodiments, the motion of the motion arm 120 may include at least one of the following: left and right deflection of the motion arm, left and right roll of the motion arm, up and down lean of the motion arm, left and right movement of the motion arm, up and down movement of the motion arm, forward and backward movement of the motion arm, and so on.

In some embodiments, the operating handle may be the master manipulator. FIG. 4 illustrates a structural schematic diagram of a master manipulator 160 according to some embodiments of the present disclosure.In some embodiments, as shown in FIG. 4, the master manipulator 160 may include a multi degree of freedom robotic arm 161 (e.g., the multi degree of freedom robotic arm 161 may include robotic arms 1611-1616) and multiple joints (e.g., joints 1601-1607) connecting the multi degree of freedom robotic arm 161. In some embodiments, the multi degree of freedom robotic arm 161 has six degrees of freedom. Each joint of the multi degree of freedom robotic arm 161 is provided with a master manipulator sensor, and joint information (such as joint angle data) is generated through the master manipulator sensor of each joint. In some embodiments, the sensors at the joints use potentiometers and/or encoders. In some embodiments, the master manipulator 160 may be provided with a control chip. The control chip may calculate the pose data of the master manipulator (such as the pose of the handle 162) according to the joint information obtained by the sensors at each joint, and send the calculated pose data to the controller. In other embodiments, the controller 140 may also calculate the pose data of the master manipulator 160 according to the joint information sent by the joint sensor.

In some embodiments, the main manipulator 160 may include a handle 162 disposed at the end of the multi degree of freedom robotic arm 161. When operating, multiple joint drivers and multiple robotic arms (such as robotic arms 1611-1616) are driven by the user to adjust the configuration of the multi degree of freedom robotic arm 161, causing the handle 162 of the multi degree of freedom robotic arm 161 to move to the appropriate pose (such as the current pose), in order to control the motion of the mobile platform 110 or the motion arm 120 through the handle 162.

In some embodiments, as shown in FIG. 3, the input device includes a handle 130 disposed on the motion arm 120. Handle 130 is configured to control the motion of motion arm 120 and mobile platform 110 and includes at least one force sensor. User input includes force and/or torque applied to the handle, and input information includes force sensor information corresponding to the force and/or torque applied to the handle.

For example, the motion arm 120 may be provided with motion arm position adjustment buttons, motion arm orientation adjustment buttons, or mobile platform adjustment buttons. The operator may select the adjustment mode by pressing different buttons. The above is only an example. The operator can also select the adjustment mode through toggle buttons, knobs, touch keys, etc., or input commands on the system input device (such as keyboard or mouse) to select the adjustment mode.

Figure 5 illustrates a coordinate diagram of the motion arm 120 of the robot system 100 according to some embodiments of the present disclosure. In some embodiments, as shown in FIG. 5, the motion arm 120 may be disposed on the crossbeam 113 of the mobile platform 110, and a joint (e.g. joint J1) may be disposed on the mobile platform 110. Joint J1 is a lifting joint used to adjust the height of the crossbeam 113, thereby adjusting the height of the motion arm 120. It should be understood that the position adjustment mode of the motion arm 120 is mainly used to adjust the position joints, such as joints J1-J3 or joints J1-J4 shown in FIG. 5, to adjust the position of the motion arm 120. It should be understood that joints 1211-1215 shown in FIG. 3 correspond to joints J2-J6 shown in FIG. 5, respectively. In position adjustment mode, joints J4-J5 of the motion arm 120 can remain unchanged. Alternatively, joints J5 and J6 of motion arm 120 can remain unchanged, and changes of joints J2 and J3 of motion arm 120 can be compensated and adjusted through joint J4 to maintain the orientation of the end of motion arm 120 unchanged. The orientation adjustment mode of the motion arm 120 is mainly used to adjust the orientation joints of the motion arm 120, such as joints J4-J6, to adjust the orientation of the motion arm 120. It should be understood that in some embodiments, the adjustment mode of the motion arm 120 may simultaneously adjust position and orientation, for example, it may be used to adjust J1-J6. In some embodiments, the motion arm 120 may include moving joints, such as a lifting joint or a telescopic joint, for adjusting the position of the motion arm 120. Joint J1 may be omitted, or joint J1 may not be adjusted during the adjustment of the orientation of the motion arm 120. For example, the motion arm 120 may include a first transverse arm 121, a second transverse arm 122, a vertical arm (not shown), a first arc arm 123, a second arc arm 124, and a third arc arm 125. The vertical arm may be disposed between the second transverse arm 122 and the first arc arm 123. In addition, the motion arm 120 may include a first transverse arm rotation joint 1211, a second transverse arm rotation joint 1212, a lifting joint, a first arc arm rotation joint 1213, a second arc arm rotation joint 1214, and a third arc arm rotation joint 1215. The lifting joint may be disposed on the vertical arm, and the first arc arm rotation joint 1213 may be disposed between the vertical arm and the first arc arm 123.

In some embodiments, as shown in FIG. 3, the arm body 123-125 of the motion arm can be an arc-shaped arm, and the proximal and distal ends of multiple arc-shaped arms are sequentially rotatably connected to form joints 1213-1215 (such as joints J4-J6 shown in FIG. 5), and the rotation axes of multiple arc-shaped arms intersect at the remote center of motion. For example, as shown in FIGS. 3 and 5, the proximal end of the arc arm 123 is rotatably connected to the distal end of the arm body 122 around the rotation axis of joint 1213 (joint J4), the proximal end of the arc arm 124 is rotatably connected to the distal end of the arc arm 123 around the rotation axis of joint 1214 (joint J5), the proximal end of the arc arm 125 is rotatably connected to the distal end of the arc arm 124 around the rotation axis of joint 1215 (joint J6), and the rotation axis of joint J4, joint J5, and joint J6 intersect at the remote center of motion (RCM) point, as shown in the origin of coordinate system {7} in FIG. 5. While keeping the RCM point stationary, the arc arms 123, 124, and 125 may move freely in the motion space, such as transforming between fully folded, fully unfolded, and intermediate positions. It should be understood that the connecting rod curvature, connecting rod thickness, and connecting rod arc length of multiple arc-shaped arms can be designed according to actual needs, so that multiple arc-shaped arms can move around the RCM point in both the unfolded and folded positions during the rotation process, and to maximize the working space of the arm body, avoiding interference between multiple arm bodies, and thereby achieving the installation of multiple surgical instruments on one motion arm. This can avoid interference between multiple motion arms when multiple motion arms are equipped with multiple surgical instruments. It should be understood that during surgery, the remote center of motion may be the abdominal entry point or incision position, and the motion arm is equipped with surgical instruments to move the surgical instruments around the RCM point for adjustment of positioning or surgical operations. The above is only an example. It should be understood that the motion arm 120 may not include an RCM mechanism composed of multiple arc-shaped arms (such as arc-shaped arms 123-125), but may include other RCM mechanisms, such as RCM mechanisms composed of multi arm bodies and multi joints, including parallelogram structures or similar structures.

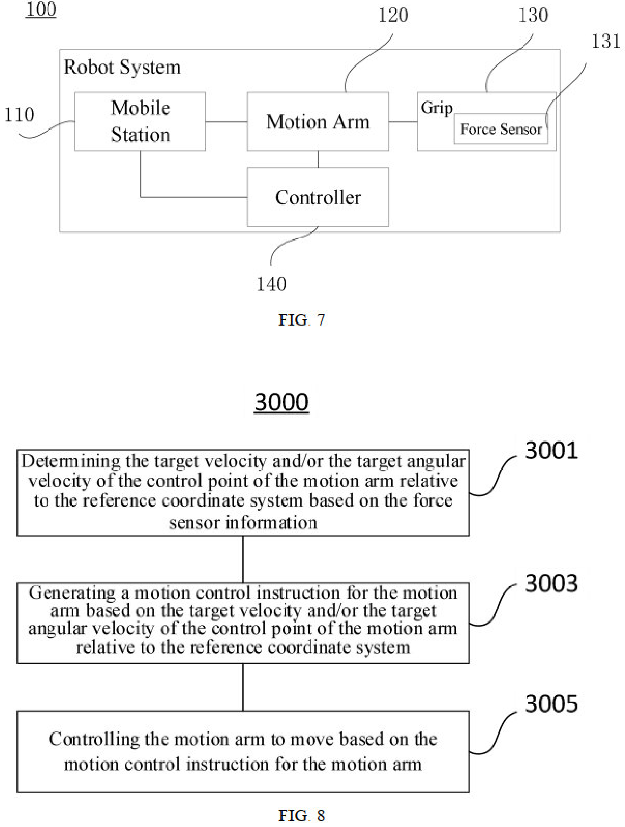

FIG. 6 illustrates a flowchart of a motion control method 2000 for a robot system 100 according to some embodiments of the present disclosure. FIG. 7 illustrates a schematic block diagram of the robot system 100 according to some embodiments of the present disclosure. In some embodiments, as shown in FIG. 7, the robot system 100 may include a mobile platform 110, at least one motion arm 120 disposed on the mobile platform 110, and a grip 130 disposed on at least one motion arm 120 (e.g., the distal end of the motion arm 120). The grip 130 may be configured to control the motion of the motion arm 120 and the mobile platform 110. The grip 130 includes at least one force sensor 131, such as a six-axis force sensor or multiple force sensors and multiple torque sensors.

During operation, the operator drags grip 130. The operator's intention to operate can be perceived based on force sensor data. The robot system 100 provides assistance to the operator to easily drag the motion arm 120 or move or lift the mobile platform 110. By dragging the motion arm 120 to move the motion arm 120 (such as the end of the arm body 125 or the control point of the motion arm 120) to the appropriate position and posture, the configuration adjustment of the motion arm 120 is achieved. The position, posture, or height of the mobile platform 110 is adjusted by dragging the mobile platform 110. In this way, preoperative positioning operations can be conveniently and easily achieved, reducing preoperative preparation time.

As shown in FIG. 6, in step 2001, the force sensor information corresponding to the force and/or torque applied to the grip is received from at least one force sensor. It should be understood that when the operator drags grip 130, for example, dragging the grip 130 along translational degrees of freedom, external force will be applied to the grip 130. Alternatively, when the grip 130 is rotated along the rotational degrees of freedom, a torque will be applied to the grip 130. In this way, the force sensor 131 disposed on the grip 130 can measure the force and/or torque applied on the grip 130 and generate force sensor information containing the corresponding force and/or torque information. In some embodiments, the force sensor information may include force vector information and/or torque vector information. For example, a force sensor may include a six-axis force sensor, wherein three axes may be used to measure force information, which is converted into force vector information through conversion (such as protocol output), and the other three axes may measure torque information, which is converted into torque vector information through conversion. Controller 140 of robot systems (such as robot system 100) may receive force sensor information from force sensors. It should be understood that the above is only an example. Force sensor 131 may also include sensors for measuring force and sensors for measuring torque.

As shown in FIG. 6, in step 2003, the motion arm or mobile platform is controlled to move based on the force sensor information. It should be understood that the controller 140 of the robot system 100 may receive force sensor information, such as force vector information and/or torque vector information, from the force sensor; control the motions of at least a portion of the joints (such as joints J1-J6) of the motion arm 120 to achieve the adjustment of the position or orientation of the motion arm 120. In some embodiments, the controller 140 of the robot system 100 may receive force sensor information, such as force vector information, from the force sensor to control the movement of the mobile platform 110, for example, for controlling the motion of the lifting joint J1 of the mobile platform 110 to achieve the ascending and descending of the mobile platform 110, or for controlling the movement of the active casters of the mobile platform 110 to achieve the forward, backward, or turning movement of the mobile platform 110.

FIG. 8 illustrates a flowchart of a motion control method 3000 of a motion arm of a robot system 100 according to some embodiments of the present disclosure. The method 3000 may be implemented or performed by hardware, software or firmware. In some embodiments, the method 3000 may be performed by a robot system (e.g., a controller 140 of the robot system 100 as shown in FIG. 2). In some embodiments, the method 3000 may be implemented as computer-readable instructions. These instructions may be read and executed by a general-purpose processor or a dedicated processor. For example, the processor of the controller 140 of the robot system 100 is configured to execute the method 3000. In some embodiments, these instructions may be stored on a computer-readable medium.

As shown in FIG. 8, in step 3001, based on the force sensor information, a target velocity and/or a target angular velocity of the control point of the motion arm relative to the reference coordinate system are determined. In some embodiments, the control point of the motion arm may include the trocar abdominal entry point, such as the remote center of motion (RCM point), RCM point may be the origin of the coordinate system {7} as shown in FIG. 5. Alternatively, the control point of the motion arm may include a trocar fixing point. For example, the trocar is fixedly connected to the end of the motion arm through a trocar fixing clip to form a trocar fixing point. In this way, the trocar fixing point may be the center of the end of the trocar fixing clip. For example, the trocar fixing point may be the origin of the coordinate system {TRB} as shown in FIG. 5. In some embodiments, the origin of the reference coordinate system, such as the coordinate system {0} shown in FIG. 5, may be located at the intersection of the ground and the proximal joint (e.g. joint J2) axis of the motion arm 120. The z-axis of the reference coordinate system {0} is vertically upward, and the x-axis is along the elongation direction of the crossbeam 113.

In some embodiments, the target velocity of the control point of the motion arm relative to the reference coordinate system {0} may be determined based on the force vector information of the force sensor. Alternatively, based on the torque vector information of the force sensor, the target angular velocity of the control point of the motion arm relative to the reference coordinate system {0} may be determined.

In some embodiments, the target velocity and/or the target angular velocity of the control point in a control point coordinate system are determined based on the force vector information and/or the torque vector information of the force sensor. The target velocity and/or the target angular velocity of the control point relative to a reference coordinate system is determined based on a target velocity and/or target angular velocity of the control point in a control point coordinate system and a transformation relationship between the control point coordinate system and the reference coordinate system. In some embodiments, determining the target velocity of the control point in the control point coordinate system may include determining the force vector information in the control point coordinate system based on the force vector information and the transformation relationship between the force sensor coordinate system and the control point coordinate system, and determining the target velocity of the control point in the control point coordinate system based on the force proportional coefficient. In some embodiments, determining the target angular velocity of the control point in the control point coordinate system may include determining the torque vector information in the control point coordinate system based on the torque vector information and the transformation relationship between the force sensor coordinate system and the control point coordinate system, and determining the target angular velocity of the control point in the control point coordinate system based on the torque proportional coefficient.

It should be understood that, as shown in FIG. 5, the reference coordinate system can be represented as {0}, and the control point coordinate system can be represented as {CP}. The RCM coordinate system can be represented as {7}, with its origin being the RCM point and z-axis pointing towards the trocar feed direction along the central axis of the trocar. The sensor coordinate system can be represented as {FT}, which is determined based on the definition of the force sensor's own coordinate system. Its pose $^{7}_{FT}T$ relative to the coordinate system {7} can be measured through a three-dimensional model. The trocar coordinate system (or, the entry point coordinate system) can be represented as {TR} (not shown), with its origin locates at the abdominal entry point and the coordinate axes direct consistently with those of the coordinate system {7}. The trocar fixing clip coordinate system can be represented as {TRB}, and its origin can be located at the center of the end of the trocar fixing clip, its coordinate axes can direct consistently with those of the coordinate system {7}. For example, {CP} can be the RCM coordinate system {7}, or it can be {TRB} or {TR} depending on the usage scenes.

For example, the force sensor information of at least one force sensor (such as a six-axis force sensor) can be represented as $f = [Fx, Fy, Fz, Mx, My, Mz]$. The force vector information expressed by the force sensor in the sensor coordinate system {FT} can be represented as $^{FT}_{FT}F = [Fx, Fy, Fz]$, the torque vector information expressed by the force sensor in the sensor coordinate system {FT} can be represented as $^{FT}_{FT}M = [Mx, My, Mz]$, the force vector information expressed by the force sensor in the control point coordinate system {CP} can be represented as $^{CP}_{FT}F = {}^{CP}_{FT}R\,{}^{FT}_{FT}F$, and the torque vector information expressed by the force sensor in the control point coordinate system {CP} can be represented as $^{CP}_{FT}M = {}^{CP}_{FT}R\,{}^{FT}_{FT}M$.

Multiplying the force vector information in the control point coordinate system {CP} by the force proportional coefficient, the target velocity of the control point in the control point coordinate system {CP} can be determined, as shown in equation (1):

$$^{CP}_{CP}v = \begin{bmatrix} k_{vx} & & \\ & k_{vy} & \\ & & k_{vz} \end{bmatrix} {}^{CP}_{FT}F \qquad \text{equation (1)}$$

Wherein, $\begin{bmatrix} k_{vx} & & \\ & k_{vy} & \\ & & k_{vz} \end{bmatrix}$ is the force proportional coefficient.

Multiplying the torque vector information in the control point coordinate system {CP} by the torque proportional coefficient, the target angular velocity of the control point in the control point coordinate system {CP} can be determined, as shown in equation (2):

$$^{CP}_{CP}\omega = \begin{bmatrix} k_{\omega x} & & \\ & k_{\omega y} & \\ & & k_{\omega z} \end{bmatrix} {}^{CP}_{FT}M \qquad \text{equation (2)}$$

Wherein, $\begin{bmatrix} k_{\omega x} & & \\ & k_{\omega y} & \\ & & k_{\omega z} \end{bmatrix}$ is the torque proportional coefficient.

Converting the target velocity of the control point in the control point coordinate system {CP} to the target velocity relative to the reference coordinate system {0}, as shown in equation (3):

$$ {}^{0}_{CP}\boldsymbol{v} = {}^{0}_{CP}R\,{}^{CP}_{CP}\boldsymbol{v} \qquad\qquad \text{equation (3)} $$

Wherein, ${}^{0}_{CP}R$ is the orientation of the control point coordinate system relative to the reference coordinate system {0}.

Converting the target angular velocity of the control point in the control point coordinate system {CP} to the target angular velocity relative to the reference coordinate system {0}, as shown in equation (4):

$$ {}^{0}_{CP}\boldsymbol{\omega} = {}^{0}_{CP}R\,{}^{CP}_{CP}\boldsymbol{\omega} \qquad\qquad \text{equation (4)} $$

As shown in FIG. 8, in step 3003, motion control instructions for the motion arm are generated based on the target velocity and/or the target angular velocity of the control point of the motion arm relative to the reference coordinate system. For example, the controller 140 of the robot system 100 may generate driving parameters for the joint motors of the motion arm 120 based on the target velocity and/or the target angular velocity, and generate motion control instructions for the motion arm 120 based on the driving parameters of the joint motors. For example, the driving parameters of joints J1-J4 may be determined based on the target velocity to generate positional motion control instructions for the motion arm. The driving parameters of joints J4-J6 may be determined based on the target angular velocity to generate orientational motion control instructions for the motion arm. The driving parameters of joints J1-J6 may be determined based on the target velocity and target angular velocity to generate pose motion control instructions for the motion arm.

As shown in FIG. 8, in step 3005, the motion arm is controlled to move based on the motion control instructions for the motion arm. It should be understood that one or more joint motors of the motion arm and/or mobile platform may be controlled based on motion control instructions to drive the motion of corresponding joints, in order to adjust the position or orientation of the motion arm. For example, joints J1-J4 may be driven to move based on positional motion control instructions. Alternatively, joints J4-J6 of the motion arm may be driven to move based on orientational motion control instructions. Alternatively, joints J1-J6 of the motion arm can be driven to move based on pose motion control instructions.

In some embodiments, the joint values and joint velocities of one or more joints may be determined based on the target velocity and/or the target angular velocity of the control point of the motion arm relative to the reference coordinate system, and the driving control instructions for one or more joint motors may be determined based on the joint values and joint velocities of the one or more joints.

The target velocity and target angular velocity of the control point relative to the reference coordinate system {0} are represented as ${}^{0}\boldsymbol{V}_{CP} = \begin{bmatrix} {}^{0}_{CP}\boldsymbol{v} \\ {}^{0}_{CP}\boldsymbol{\omega} \end{bmatrix}$. Using the differential kinematics model, based on equation (5), it can be concluded that:

$$ {}^{0}\boldsymbol{V}_{CP} = \begin{bmatrix} {}^{0}\boldsymbol{v}_{CP} \\ {}^{0}\boldsymbol{\omega}_{CP} \end{bmatrix} = \begin{bmatrix} J_{v1} & J_{v2} & J_{v3} & J_{v4} & J_{v5} & J_{v6} \\ J_{w1} & J_{w2} & J_{w3} & J_{w4} & J_{w5} & J_{w6} \end{bmatrix} [\dot{q}_1 \quad \dot{q}_2 \quad \dot{q}_3 \quad \dot{q}_4 \quad \dot{q}_5 \quad \dot{q}_6]^T = J\dot{q} $$

$$ \text{equation (5)} $$

Wherein $J_{vi}$ represents the partial derivative of the control point velocity vector with respect to the i-th joint velocity, $J_{wi}$ represents the partial derivative of the control point coordinate system angular velocity vector with respect to the i-th joint velocity, and $\dot{q}_i$ represents the joint velocity of the i-th joint. For example, $J_{v1}$ represents the partial derivative of the control point velocity vector with respect to the joint velocity of the lift joint J1, $J_{w1}$ represents the partial derivative of the control point coordinate system angular velocity vector with respect to the joint velocity of the lift joint J1 shown in FIG. 5, $J_{v2}$ represents the partial derivative of the control point velocity vector with respect to the joint velocity of the first transverse arm rotation joint 1211 (joint J2 shown in FIG. 5) shown in FIG. 3, $J_{w2}$ represents the partial derivative of the control point coordinate system angular velocity vector with respect to the joint velocity of the first transverse arm rotation joint 1211 (joint J2), $\dot{q}_1$ represents the joint velocity of the lift joint J1, $\dot{q}_2$ represents the joint velocity of the first transverse arm rotation joint 1211 (joint J2), and so on for other joints.

After synthesizing the six dimensional velocity requirements through equations (1) to (4), the target joint velocity can be calculated using Jacobian pseudo inverse and further converted into joint angle instructions.

Determining the joint values and joint velocities of one or more joints are shown in equations (6) and (7):

$$\dot{q} = J^\dagger \, {}^0V_{CP} \qquad\qquad \text{equation (6)}$$

$$q = q + \dot{q}dt \qquad\qquad \text{equation (7)}$$

Wherein $q$ is the joint value vector of one or more joints, $\dot{q}$ is the joint velocity vector of one or more joints, and $J^\dagger$ is the pseudo inverse matrix of the Jacobian matrix J.

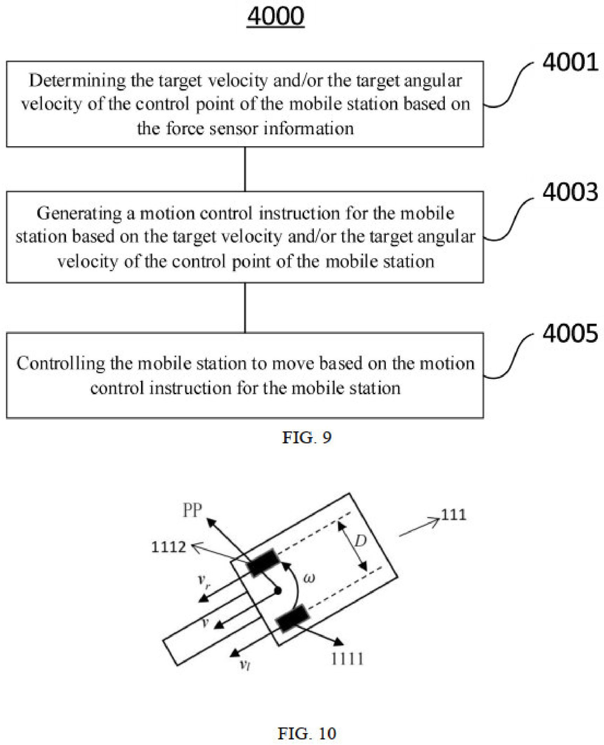

FIG. 9 illustrates a flowchart of a motion control method 4000 of a mobile platform of a robot system according to some embodiments of the present disclosure. The method 4000 may be implemented or performed by hardware, software or firmware. In some embodiments, the method 4000 may be performed by a robot system (e.g., a controller 140 of the robot system 100 as shown in FIG. 2). In some embodiments, the method 4000 may be implemented as computer-readable instructions. These instructions may be read and executed by a general-purpose processor or a dedicated processor. For example, the processor of the controller 140 of the robot system 100 is configured to execute the method 4000. In some embodiments, these instructions may be stored on a computer-readable medium.

As shown in FIG. 9, in step 4001, the target velocity and/or the target angular velocity of the control point of the mobile platform are determined based on the force sensor information. In some embodiments, the force sensor information includes force vector information. In some embodiments, one or more active casters may include first and second casters that are disposed symmetrically. FIG. 10 illustrates a simplified schematic diagram of a control point of the mobile platform 110 of the robot system 100 according to some embodiments of the present disclosure. For example, the mobile platform 110 may include two active casters and two passive casters, with the two active casters symmetrically arranged (such as the front left casters 1111 and right casters 1112 shown in FIG. 10) and the two passive casters symmetrically arranged (not shown in the figure). It should be understood that the positions of the two active casters and the two passive casters may be interchanged with each other.

It should be understood that the control point PP of the mobile platform 110 can be the midpoint of two active casters 1111 and 1112. In some embodiments, as shown in FIG. 5, the mobile platform 110 may further include a column 112, a lifting joint J1 connected to the column 112, and a column lifting motor driving the lifting joint J1. The target velocity of the control point PP of the mobile platform may include the active caster target forward velocity and/or target lifting velocity. The target angular velocity of the control point PP of the mobile platform may include the mobile platform target turning velocity.

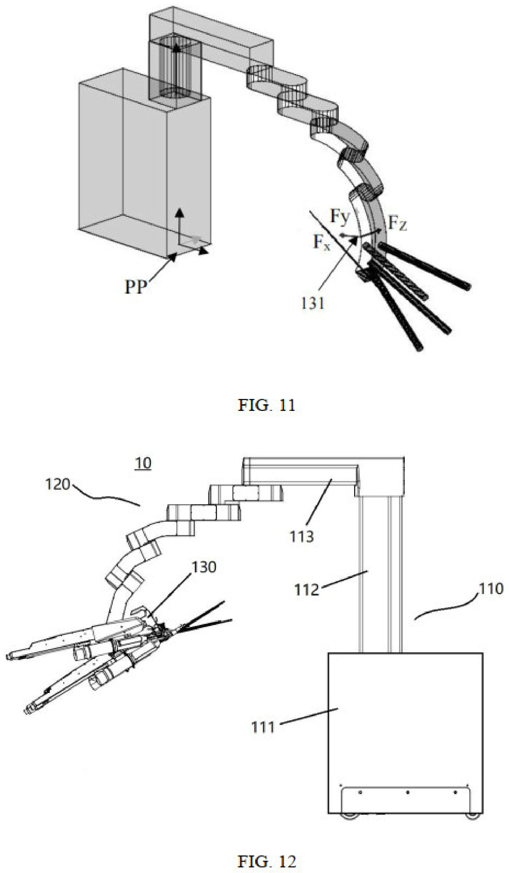

In some embodiments, the force vector information in the mobile platform control point coordinate system may be determined based on the force vector information and the transformation relationship between the force sensor coordinate system and the mobile platform control point coordinate system. In some embodiments, the torque vector information in the mobile platform control point coordinate system may be determined based on the force vector information in the mobile platform control point coordinate system. FIG. 11 illustrates a schematic diagram of a control point coordinate of the mobile platform 110 of the robot system 100 according to some embodiments of the present disclosure. As shown in FIG. 11, the mobile platform control point coordinate system can be represented as {PP}. The vertical height of the origin of the mobile platform control point coordinate system {PP} can be consistent with the reference coordinate system, such as the coordinate system {0}. The horizontal position of the origin of the coordinate system {PP} is projected on the ground as the projection of the midpoint of the two active casters on the ground. The coordinate axis directions of {PP} are consistent with those of coordinate system {0}, and it is a translation of coordinate system {0} along the x-axis.

For example, the target velocity and/or target angular velocity of the control point of the mobile platform may be calculated based on equation (8):

$$\begin{bmatrix} v \\ \omega \end{bmatrix} = \begin{bmatrix} 1/2 & 1/2 \\ 1/D & -1/D \end{bmatrix} \begin{bmatrix} v_r \\ v_l \end{bmatrix} \qquad\qquad \text{equation (8)}$$

Wherein, the forward velocity of the patient-side cart is denoted as $v$, directional angular velocity of the patient-side cart is denoted as $\omega$, left wheel velocity of the patient-side cart is denoted as $v_l$, right wheel velocity of the patient-side cart is denoted as $v_r$, and distance between the two active casters of the patient-side cart is donated as $D$.

In some embodiments, for a given target velocity and target angular velocity of a mobile platform, the velocity of the two active casters can be calculated based on equation (9):

$$\begin{bmatrix} v_r \\ v_l \end{bmatrix} = \begin{bmatrix} 1/2 & 1/2 \\ 1/D & -1/D \end{bmatrix}^{-1} \begin{bmatrix} v \\ \omega \end{bmatrix} = \begin{bmatrix} 1 & D/2 \\ 1 & -D/2 \end{bmatrix} \begin{bmatrix} v \\ \omega \end{bmatrix} \qquad \text{equation (9)}$$

In some embodiments, the pose of the force sensor coordinate system {FT} relative to {PP} can be calculated based on the joint values of the motion arm, as shown in equation (10):

$$^{PP}_{FT}T = {}^{PP}_{0}T\,{}^{0}_{1}T\,{}^{1}_{2}T\,{}^{2}_{3}T\,{}^{3}_{4}T\,{}^{4}_{5}T\,{}^{5}_{6}T\,{}^{6}_{7}T\,{}^{7}_{FT}T = \begin{bmatrix} {}^{PP}_{FT}R & {}^{PP}_{FT}p \\ \mathbf{0} & 1 \end{bmatrix} \qquad \text{equation (10)}$$

For example, $^{n}_{m}T$ represents the pose of the coordinate system m relative to the coordinate system n; $^{n}_{m}R$ represents the orientation of the coordinate system m relative to the coordinate system n; and $^{n}_{m}p$ represents the position of the coordinate system m relative to the coordinate system n.

Referring to the arm bodies and joints shown in FIGS. 3 and 5, the relevant coordinate systems are defined as follows:

Coordinate system {1} has its origin located at the intersection of the lower surface of the crossbeam and the J2 axis of the joint, with the z-axis pointing vertically upward and the x-axis extending along the length of the crossbeam. Coordinate system {1} is a translation of coordinate system {0} along the z-axis;

Coordinate system {2} has its origin coinciding with the origin of coordinate system {1}, with the z-axis pointing vertically upward along joint axis J2 and the x-axis pointing from the first transverse arm rotation joint J2 to joint J3; Coordinate system {2} is a rotation of coordinate system {1} about the z-axis;

Coordinate system {3} has its origin located at the intersection of joint J3 axis and the upper surface of the second transverse arm 122, with the z-axis pointing vertically upward along joint J3 axis, the x-axis pointing from joint J3 to joint J4 along the direction of second transverse arm 122. Coordinate system {3} is a translation of coordinate system {2} along the x-axis and the z-axis, and then rotated around the z-axis;

Coordinate system {4} has its origin located at the RCM point, with the z-axis pointing vertically upward along the joint J4 axis and the x-axis being perpendicular to the plane where the center arc of the first arc arm 123 is located, pointing to the direction of z5 cross-products z4. Coordinate system {4} is a translation of coordinate system {3} along the x-axis and the z-axis, and then rotated around the z-axis;

Coordinate system {5} has its origin at the RCM point, with the z-axis pointing upwards along the joint J5 axis and the x-axis being perpendicular to the plane where the center arc of the second arc arm 124 is located, pointing to the direction of z6 cross-products z5. Coordinate system {5} is a rotation of coordinate system {4} around the x-axis and then around the z-axis;

Coordinate system {6} has its origin at the RCM point, with the z-axis pointing upwards along the joint J6 axis and the x-axis being perpendicular to the plane where the center arc of the second arc arm 125 is located, pointing to the direction of z6 cross-products z7. Coordinate system {6} is a rotation of coordinate system {5} around the x-axis and then around the z-axis;

Coordinate system {7} has its origin at the RCM point, with the z-axis pointing along the central axis of the trocar towards the direction of trocar feeding, and the x-axis pointing to the same direction as x6. Coordinate system {7} is a rotation of coordinate system {6} around the x-axis;

For example, $^{PP}_{0}T$ represents the pose of the reference coordinate system {0} relative to the mobile platform control point coordinate system {PP}, $^{0}_{1}T$ represents the pose of the coordinate system {1} relative to the reference coordinate system {0}, and so on. $^{PP}_{FT}R$ $^{PP}_{FT}R$ Represents the orientation of the sensor coordinate system {FT} relative to the mobile platform control point coordinate system {PP}, and $^{PP}_{FT}p$ represents the position of the sensor coordinate system {FT} relative to the mobile platform control point coordinate system {PP}. The force sensor information of a force sensor (such as a six-axis force sensor) may be represented as $f = [Fx, Fy, Fz, Mx, My, Mz]$. The force vector information expressed by the force sensor in the coordinate system {FT} may be represented as

$$\substack{FT\\FT}F = \begin{bmatrix} Fx, Fy, Fz \end{bmatrix}$$, and the force vector information expressed by the force sensor in the coordinate system {PP} may be represented as $\substack{PP\\FT}F = \substack{PP\\FT}R\,\substack{FT\\FT}F$. It should be understood that the force vector $\substack{PP\\FT}F$ may generate a torque vector relative to the coordinate system {PP} at the position $\substack{PP\\FT}p$, expressed as $\substack{PP\\FT}M = \substack{PP\\FT}p \times \substack{PP\\FT}F$.

In some embodiments, the force in the forward direction of the mobile platform is determined based on the forward (e.g., x-axis) directional component of the force vector information in the control point coordinate system of the mobile platform. The target forward velocity of the mobile platform is determined based on the forward force proportional coefficient. In some embodiments, the torque of the turning of the mobile platform is determined based on the vertical (e.g. z-axis) directional component of the torque vector information in the mobile platform control point coordinate system. The target angular velocity of the mobile platform is determined based on the turning torque proportional coefficient. In some embodiments, the force in the lifting direction of the mobile platform is determined based on the vertical (e.g. z-axis) directional component of the force vector information in the mobile platform control point coordinate system. The target lifting velocity of the mobile platform is determined based on the lifting proportional coefficient.

For example, the x-axis component in the force vector $\substack{PP\\FT}F$ is the force $F_{fwd}$ along the forward direction of the mobile platform, the z-axis component in the torque vector $\substack{PP\\FT}M$ is the torque $M_{rot}$ that turns the mobile platform, and the z-axis component in the force vector $\substack{PP\\FT}F$ is the force $F_{ud}$ along the lifting direction of the mobile platform.

Multiplying $F_{fwd}$ by the forward force proportional coefficient $k_v$, the target velocity $v_{tgt}$ of the mobile platform may be determined; multiplying $M_{rot}$ by the turning torque proportional coefficient $k_\omega$, the target angular velocity $\omega_{tgt}$ of the mobile platform may be determined; and multiplying $F_{ud}$ by the lift proportional coefficient $k_w$, the target lift velocity $w_{tgt}$ of the mobile platform may be determined, as shown in equations (11), (12), and (13):

$$v_{tgt} = k_v F_{fwd} \qquad\qquad \text{equation (11)}$$

$$\omega_{tgt} = k_\omega M_{rot} \qquad\qquad \text{equation (12)}$$

$$w_{tgt} = k_w F_{ud} \qquad\qquad \text{equation (13)}$$

As shown in FIG. 9, in step 4003, motion control instructions for the mobile platform are generated based on the target velocity and/or the target angular velocity of the control point of the mobile platform. In some embodiments, the target velocity of one or more active casters may be determined based on the target forward velocity of the mobile platform and the target angular velocity of the mobile platform. By using active caster motion control instructions, the motions of the active casters are driven, so as to drive the mobile platform moving forward. By using column motion control commands, the motions of the column lifting motors are driven, so as to drive the mobile platform ascending and descending.

For example, the target velocities of the left and right active casters can be determined according to equation (14):

$$\begin{bmatrix} v_{rtgt} \\ v_{ltgt} \end{bmatrix} = \begin{bmatrix} 1 & D/2 \\ 1 & -D/2 \end{bmatrix} \begin{bmatrix} v_{tgt} \\ \omega_{tgt} \end{bmatrix} \qquad\qquad \text{equation (14)}$$

Wherein, $v_{rtgt}$ represents the target velocity of the right active caster and $v_{ltgt}$ represents the target velocity of the left active caster.

In some embodiments, driving control instructions for one or more active caster motors are determined based on the target velocities and radii of the one or more active casters. It should be understood that the target velocity and radius of the active caster can be converted into the RPM instruction (e.g. revolutions per minute instruction) for the active caster, and then converted into the RPM instruction for the active caster motor based on the transmission ratio. According to equations (15) and (16), the RPM instruction for the active caster may be determined as follows:

$$rpm_{l\_tgt} = v_{l\_tgt}/r_{wheel}\frac{60}{2\pi} \qquad\qquad \text{equation (15)}$$

$$rpm_{r\_tgt} = v_{r\_tgt}/r_{wheel}\frac{60}{2\pi} \qquad\qquad \text{equation (16)}$$

Wherein: $rpm_{l\_tgt}$ represents the RPM instruction of the left active caster, $v_{l\_tgt}$ represents the target linear velocity of the left active caster relative to the ground, $rpm_{r\_tgt}$ represents the RPM instruction of the right active caster, $v_{r\_tgt}$ represents the target linear velocity of the right active caster relative to the ground, and $r_{wheel}$ represents the caster radius.

In some embodiments, the driving control instruction for the column lifting motor is determined based on the target lifting velocity of the mobile platform. It should be understood that based on the target lifting velocity, the RPM instruction of the column lifting motor may be calculated according to the lead and transmission ratio so as to control the lifting of the mobile platform.

As shown in FIG. 9, in step 4005, the mobile platform is controlled to move based on the motion control instructions for the mobile platform. For example, the forward, backward, or turning motion of the active caster may be controlled based on the RPM instruction of the active caster motor. The ascending and descending motions of the column may be controlled based on the RPM instruction of the column lifting motor, so as to perform ascending and descending of the mobile platform.

In some embodiments, the control method 1000 or 2000 further includes detecting whether the motion arm is at the limit of the motion range or whether the motion arm may collide, and in response to the motion arm being at the limit of the motion range or the motion arm possibly colliding, controlling the motion arm to stop moving or issuing an alarm message. In some embodiments, the limit of the motion range includes the motion limit of each joint in one or more joints of the motion arm. It should be understood that there are sensors (such as encoders or potentiometers) at each joint of the motion arm 120. Joint information (such as joint angle data) is generated by the sensors of each joint. The surgical robot system 100 (such as controller 140) detects joint angle data of the sensors to determine whether the motion arm 120 is at the limit of motion range. For example, the limit of motion range of a joint may be the mechanical limit of motion range of each joint (e.g. joints 1211-1215) of the motion arm 120 or the limit of motion angle set by the system. It should be understood that the distance between one or more collision points on the arm body that are prone to collision of the motion arm can be determined through the motion arm model (for example, the collision between the linear module 126 at the end of the arm body 125 of the motion arm 120 and the arm body 121 to the arm body 125, or the collision between the linear module 126 of the motion arm 120 and the column 112 or beam 113), in order to determine whether the motion arm 120 is likely to collide. In some embodiments, the alarm information may include but is not limited to: interface prompts, voice prompts, traffic light prompts, etc.

In some embodiments, the mobile platform or motion arm can be controlled to stop the motion based on the detection of the disappearance of force or torque applied to the grip. For example, the operator releases the handle 130 after dragging the motion arm 120 or the mobile platform 110 to the target pose, so that the force or torque applied to the handle 130 disappears. The surgical robot system (such as the controller 140) detects the disappearance of force or torque and controls the mobile platform 110 or the motion arm 120 to stop the motion.

FIG. 12 illustrates a structural schematic diagram of a surgical robot 10 according to some embodiments of the present disclosure. In some embodiments, as shown in FIG. 12, a surgical robot 10 includes a mobile platform 110, a motion arm 120, a grip 130, and a controller. The motion arm 120 is flexibly disposed on the mobile platform 110, and the grip 130 is disposed on the motion arm 120 (e.g., the distal end of the motion arm 120) and configured to control the motion of the motion arm 120 and the mobile platform 110. The grip 130 includes at least one force sensor 131, the force sensor 131 is used to detect the force or torque externally applied to the grip 130. The controller (e.g., controller 140) is configured to execute any of the control methods (e.g., control methods 1000, 2000, or 3000) described in the present disclosure.

In some embodiments, as shown in FIG. 12, the mobile platform 110 may include a base 111, a column 112 disposed on the base 111, a lifting joint (e.g. joint J1 shown in FIG. 5) connected to the column 112, a column lifting motor driving the column lifting joint, and a crossbeam 113 disposed on the column 112. The base 111 includes one or more active casters and one or more active caster motors that drive the active casters.

In some embodiments, as shown in FIG. 2, the controller (such as controller 140) may be communicatively connected to the motion arm 120 and the mobile platform 110, such as through cable connection or wireless connection, to achieve communication between the motion arm 120 and the mobile platform 110. Controller 140 may be configured on a computer device and disposed inside the mobile platform 110. The base 111 of the mobile platform 110 is used to achieve the moving and positioning functions of the mobile platform 110. The base 111 of the mobile platform 110 is used to integrate mobile platform electrical components internally. Column 112 may be ascended or descended, and its top is fixed to crossbeam 113. The end portion of the crossbeam 113 is connected to a motion arm 120. In some embodiments, the motion arm 120 may include one or more joints of one or more arm bodies, as well as one or more joint motors driving the one or more joints. The arm bodies include end arm bodies. It should be understood that at least one motion arm 120 may also include multiple motion arms.

In some embodiments, grip 130 further includes a main body fixedly disposed on the end terminal arm body 125 of the motion arm 120. The force sensor comprises a six-axis force sensor, which is disposed inside the main body and used to detect the force or torque externally applied to the handle 130.

In some embodiments, the present disclosure provides a computer storage medium. The storage medium may include at least one instruction, the at least one instruction, when executed by a processor, configures the processor to perform the control method described in any of the above embodiments.

In some embodiments, the present disclosure provides a computer system, which may include a storage medium and a processor. The storage medium includes at least one instruction. The processor is configured to execute the at least one instruction to configure the processor to perform the control method in any of the above embodiments.

Based on the above description of the implementations, those skilled in the art can clearly understand that the present disclosure can be implemented with the aid of software and necessary general-purpose hardware, and of course, it can also be implemented through hardware. Based on such understanding, the technical solution disclosed in the present disclosure, or the part that contributes to the existing technology, can be embodied in the form of a software product. The computer software product can be stored in a computer-readable storage medium, such as a computer floppy disk, Read-Only Memory (ROM), Random Access Memory (RAM), Flash memory (FLASH), hard disk, or optical disc, and includes several instructions to enable a computer device (which can be a personal computer, server, or network device) to execute the methods described in various embodiments of the present disclosure.

This disclosure also discloses the following embodiments:

Item 1: A motion control method for a robot system, the robot system comprising a mobile platform, at least one motion arm disposed on the mobile platform, and a handle disposed on the motion arm, the handle being configured to control the motion of the motion arm and the mobile platform and comprising at least one force sensor, the control method comprises:

receiving, from the at least one force sensor, force sensor information corresponding to the force and/or the torque applied to the grip; and
controlling, based on the force sensor information, the motion arm or the mobile platform to move.

Item 2: the control method according to item 1, the force sensor information comprises force vector information and/or torque vector information,
the control method further comprises:

determining, based on the force sensor information, a target velocity and/or a target angular velocity of a control point of the motion arm relative to a reference coordinate system;
generating, based on the target velocity and/or the target angular velocity of the control points of the motion arm relative to the reference coordinate system, motion control instructions for the motion arm; and
controlling, based on the motion control instructions for the motion arm, the motion arm to move; or
the control method further comprises:

determining, based on the force sensor information, a target velocity and/or a target angular velocity of a control point of the mobile platform;
generating, based on the target velocity and/or the target angular velocity of the control point of the mobile platform, motion control instructions for the mobile platform; and
controlling, based on the motion control instructions for the mobile platform, the mobile platform to move.

Item 3: The control method according to item 1 or item 2, further comprises:
receive the selection of the motion arm adjustment mode or the mobile platform adjustment mode.
Item 4: The control method according to item 2, the method comprises:

determining, based on force vector information of the force sensor, a target velocity of a control point of the motion arm relative to a reference coordinate system; and/or
determining, based on torque vector information of the force sensor, a target angular velocity of a control point of the motion arm relative to a reference coordinate system.

Item 5: The control method according to item 4, the method comprises:

determining, based on force vector information and/or torque vector information of the force sensor, a target velocity and/or a target angular velocity of the control point in a control point coordinate system; and
determining, based on a target velocity and/or a target angular velocity of the control point in a control point coordinate system and a transformation relationship between the control point coordinate system and the reference coordinate system, a target velocity and/or a target angular velocity of the control point relative to a reference coordinate system.

Item 6: the control method according to item 5, determining a target velocity and/or a angular velocity of the control point in a control point coordinate system comprises:

determining, based on the force vector information and the transformation relationship between a force sensor coordinate system and the control point coordinate system, force vector information in a control point coordinate system; and
determining, based on a force proportional coefficient, a target velocity of the control point in a control point coordinate system; and/or
determining, based on the torque vector information and the transformation relationship between a force sensor coordinate system and the control point coordinate system, torque vector information in a control point coordinate system; and
determining, based on a torque proportional coefficient, a target angular velocity of the control point in a control point coordinate system.

Item 7: The control method according to item 5 or item 6,

the control point coordinate system comprises a trocar coordinate system, a trocar fixing point coordinate system or remote center of motion (RCM) coordinate system; and/or
an origin of the reference coordinate system is located at an intersection of a ground and a proximal joint axis of the motion arm.

Item 8: the control method according to any one of items 2-7, the motion arm, or the motion arm and the mobile platform, comprise one or more joints and one or more joint motors driving the one or more joints, generating motion control instructions for the motion arm comprises:

determining, based on a target velocity and/or a target angular velocity of a control point of the motion arm relative to the reference coordinate system, joint values and joint velocities of the one or more joints; and
determining, based on joint values and joint velocities of one or more joints, driving control instructions for the one or more joint motors.

Item 9: The control method according to item 8, the one or more joints comprises:

a lifting joint of the mobile platform; and
a first transverse arm rotation joint, a second transverse arm rotation joint, a first arc arm rotation joint, a second arc arm rotation joint and a third arc arm rotation joint of the motion arm; or
the one or more joints comprise:
a first transverse arm rotation joint, a second transverse arm rotation joint, a lifting joint, a first arc arm rotation joint, a second arc arm rotation joint and a third arc arm rotation joint of the motion arm.

Item 10: the control method according to any one of items 2-9, the method comprises:

determining, based on the force vector information and a transformation relationship between a force sensor coordinate system and the mobile platform control point coordinate system, force vector information in the mobile platform control point coordinate system; and/or
determining, based on force vector information in the mobile platform control point coordinate system, torque vector information in the mobile platform control point coordinate system.

Item 11: the control method according to item 10, determining a target velocity and/or a target angular velocity of a control point of the mobile platform comprises:

determining, based on a forward direction component of force vector information in the mobile platform control point coordinate system, a force in a forward direction of the mobile platform; and

determining, based on a forward force proportional coefficient, a target forward velocity of the mobile platform; and/or

determining, based on a vertical direction component of torque vector information in the mobile platform control point coordinate system, a torque of turning of the mobile platform; and

determining, based on a turning torque proportional coefficient, a target angular velocity of the mobile platform; and/or

determining, based on a vertical direction component of force vector information in the mobile platform control point coordinate system, a force in a lifting direction of the mobile platform; and

determining, based on a lifting proportional coefficient, a target lifting velocity of the mobile platform.

Item 12: the control method according to item 11, the mobile platform comprises one or more active casters and one or more caster motors driving the active casters, the method comprises:

determining, based on a target forward velocity of the mobile platform and a target angular velocity of the mobile platform, target velocities of the one or more active casters; and

determining, based on target velocities and radii of the one or more active casters, driving control instructions for the one or more active caster motors; and/or

the mobile platform comprises a column, a lifting joint connected with the column and a column lifting motor driving the column lifting joint, the method comprises:

determining, based on a target lifting velocity of the mobile platform, driving control instructions for the column lifting motor.

Item 13: The control method according to item 12,

the active casters comprise a first caster and a second caster that are disposed symmetrically;

a vertical height of an origin of the mobile platform control point coordinate system is the same as a vertical height of the reference coordinate system, a horizontal position of an origin of the mobile platform control point coordinate system is projected within the ground as the projection of a midpoint of the first caster and the second caster on the ground.

Item 14: the control method according to any one of items 1-13, further comprises:

detecting whether the motion arm is at the limit of motion range or whether there is a possibility of collision of the motion arm;

in response to the motion arm being at the limit of motion range or the possibility of collision of the motion arm, control the motion arm to stop the motion or issue an alarm message.

Item 15: the control method according to any one of items 1-14, further comprises:
based on the detection of disappearance of the force or torque applied to the handle, controlling the mobile platform or the motion arm to stop the motion.
Item 16: a surgical robot, comprises:

a mobile platform;
a motion arm, the motion arm is movably disposed on the mobile platform;
a grip disposed on the motion arm and configured to control motions of the motion arm and the mobile platform, the grip comprises at least one force sensor, the force sensor is used to detect a force or torque externally applied to the grip; and
a controller configured to execute the control method according to any one of items 1-15.

Item 17: the surgical robot according to Item 16,

the motion arm comprises one or more arm bodies, the arm body includes an end terminal arm body;
the grip further comprises a main body, the main body is fixedly disposed on the end terminal arm body of the motion arm, the at least one force sensor comprises a six-axis force sensor, the six-axis force sensor is disposed inside the main body and used to detect the force or torque externally applied to the grip.
Item 18: the surgical robot according to item 17, the motion arm further comprises one or more joints connecting the one or more arm bodies, and one or more joint motors driving the one or more joints;
the mobile platform comprises a base, a column disposed on the base, a lifting joint connected to the column, a column lifting motor driving the column lifting joint, and a crossbeam disposed on the column, the base comprises one or more active casters and one or more active caster motors driving the active casters.

Item 19: a computer storage medium, the storage medium comprises at least one instruction, the at least one instruction is executed by a processor to configure the processor to execute the control method according to any one of items 1-15.

Item 20: a surgical robot, comprises:

a storage medium comprising at least one instruction; and
a processor configured to execute the at least one instruction to configure the processor to execute the control method according to any one of items 1-15.

Item 21: a motion control method for a robot system, the robot system comprises a mobile platform, at least one motion arm disposed on the mobile platform, and an input device, the input device is configured to control a motion of the motion arm and the mobile platform, the control method comprises:

receiving, from the input device, input information corresponding to user input; and
controlling, based on the input information, the motion arm or the mobile platform to move.

Item 22: the control method according to item 21, the input device comprises at least one of: a grip disposed on the motion arm, an operating handle, an input interface or at least one mechanical button.

Item 23: the control method according to claim 22, the input device comprises a grip disposed on the motion arm, the grip is configured to control the motion of the motion arm and the mobile platform, and the grip comprises at least one force sensor, the user input comprises a force and/or a torque applied to the grip, the input information comprises force sensor information corresponding to the force and/or the torque applied to the grip, the control method comprises:

receiving, from the at least one force sensor, force sensor information corresponding to the force and/or the torque applied to the grip; and
controlling, based on the force sensor information, the motion arm or the mobile platform to move.

Item 24: the control method according to item 23, the force sensor information comprises force vector information and/or torque vector information, the control method further comprises:

determining, based on the force sensor information, a target velocity and/or a target angular velocity of a control point of the motion arm relative to a reference coordinate system;
generating, based on the target velocity and/or the target angular velocity of the control points of the motion arm relative to the reference coordinate system, motion control instructions for the motion arm; and
controlling, based on the motion control instructions for the motion arm, the motion arm to move; or
the control method further comprises:

determining, based on the force sensor information, a target velocity and/or a target angular velocity of a control point of the mobile platform;
generating, based on the target velocity and/or the target angular velocity of the control point of the mobile platform, motion control instructions for the mobile platform; and
controlling, based on the motion control instructions for the mobile platform, the mobile platform to move.

Item 25: The control method according to Item 24, the method comprises:

determining, based on the force vector information, a target velocity of a control point of the motion arm relative to a reference coordinate system; and/or
determining, based on torque vector information of the force sensor, a target angular velocity of a control point of the motion arm relative to a reference coordinate system.

Item 26: the control method according to any one of items 21-25, further comprises:

detecting whether the motion arm is at the limit of motion range or whether there is a possibility of collision with the motion arm;
in response to the motion arm being at the limit of motion range or the possibility of collision of the motion arm, control the motion arm to stop the motion or issue an alarm message.

Item 27: the control method according to any one of items 23-26, further comprises:
based on the detection of disappearance of the force or torque applied to the handle, controlling the mobile platform or the motion arm to stop the motion.
Item 28: a surgical robot, comprises:

a mobile platform;
a motion arm, the motion arm is movably disposed on the mobile platform;
a grip disposed on the motion arm and configured to control motions of the motion arm and the mobile platform, the grip comprises at least one force sensor, the force sensor is used to detect a force or torque externally applied to the grip; and
a controller configured to execute the control method according to any one of items 21-27.

Item 29: the surgical robot according to Item 28,

the motion arm comprises one or more arm bodies, the arm body includes an end terminal arm body;
the grip further comprises a main body, the main body is fixedly disposed on the end terminal arm body of the motion arm, the at least one force sensor comprises a six-axis force sensor, the six-axis force sensor is disposed inside the main body and used to detect the force or torque externally applied to the grip.

Item 30: the surgical robot according to item 29, the motion arm further comprises one or more joints connecting the one or more arm bodies, and one or more joint motors driving the one or more joints;
the mobile platform comprises a base, a column disposed on the base, a lifting joint connected to the column, a column lifting motor driving the column lifting joint, and a crossbeam disposed on the column, the base comprises one or more active casters and one or more active caster motors driving the active casters.

Note that the above are only exemplary embodiments of the present disclosure and the applied technical principles. Those skilled in the art will appreciate that the present disclosure is not limited to specific embodiments herein, and those skilled in the art can make various apparent changes, readjustments and substitutions without departing from the scope of protection of the present disclosure. Thus, although the present disclosure is described in more detail by the above embodiments, the present disclosure is not limited to the above embodiments. Without departing from the concept of the present disclosure, more other equivalent embodiments may be included, and the scope of the present disclosure is determined by the scope of the appended claims.

## Claims

1. A motion control method for a robot system, wherein the robot system comprises a mobile platform, at least one motion arm disposed on the mobile platform, and an input device, the input device is used to control a motion of the motion arm and the mobile platform, the control method comprises:

   receiving, from the input device, input information corresponding to user input; and
   controlling, based on the input information, the motion arm or the mobile platform to move.

2. The control method according to claim 1, wherein the input device comprises at least one of: a grip disposed on the motion arm, an operating handle, an input interface, or at least one mechanical button.

3. The control method according to claim 2, wherein the input device comprises a motion arm adjustment mode selection key or a mobile platform adjustment mode selection key;

   the motion arm adjustment mode selection key comprises a motion arm position adjustment mode selection key and a motion arm orientation adjustment mode selection key;
   The control method comprises:
   receiving the selection of the motion arm adjustment mode or the mobile platform adjustment mode.

4. The control method according to claim 2 or 3, wherein the operating handle is configured to move in at least two dimensions; or

the input interface comprises input keys configured to adjust in at least two dimensions; or
the at least one mechanical button comprises multiple buttons configured to adjust in at least two dimensions.

5. The control method according to claim 2 or 3, wherein the input device comprises a grip disposed on the motion arm, the grip is used to control the motion of the motion arm and the mobile platform, and the grip comprises at least one force sensor, the user input comprises a force and/or a torque applied to the grip, the input information comprises force sensor information corresponding to the force and/or the torque applied to the grip, the control method comprises:

receiving, from the at least one force sensor, force sensor information corresponding to the force and/or the torque applied to the grip; and
controlling, based on the force sensor information, the motion arm or the mobile platform to move.

6. The control method according to any one of claims 2-4,
wherein the input device comprises an operating handle, the user input comprises an operating action to the operating handle, the input information comprises handle operating information, the control method comprises:

receiving, from the operating handle, handle operating information corresponding to the operating action to the operating handle; and
controlling, based on the handle operating information, the motion arm or the mobile platform to move; or
the input device comprises an input interface, the user input comprises operating action to the input interface, the input information comprises interface operating information, the control method comprises:

receiving, from the input interface, the interface operating information corresponding to the operating action to the input interface; and
controlling, based on the interface operating information, the motion arm or the mobile platform to move; or
the input device comprises a mechanical button, the user input comprises operating action to the mechanical button, the input information comprises button operating information, the control method comprises:

receiving, from the mechanical button, the button operating information corresponding to the operating action to the mechanical button; and
controlling, based on the button operating information, the motion arm or the mobile platform to move.

7. The control method according to any one of claims 1-6, wherein the motion of the mobile platform comprises at least one of: mobile platform moving forward and backward, mobile platform turning left and right, or mobile platform ascending and descending; or
the motion of the motion arm comprises at least one of: the motion arm deflects left and right, the motion arm rolls left and right, the motion arm leans up and down, the motion arm moves left and right, the motion arm moves up and down, or the motion arm moves forward and backward.

8. The control method according to claim 5, wherein the force sensor information comprises force vector information and/or torque vector information,
the control method further comprises:

determining, based on the force sensor information, a target velocity and/or a target angular velocity of a control point of the motion arm relative to a reference coordinate system;
generating, based on the target velocity and/or the target angular velocity of the control points of the motion arm relative to the reference coordinate system, motion control instructions for the motion arm; and
controlling, based on the motion control instructions for the motion arm, the motion arm to move; or
the control method further comprises:

determining, based on the force sensor information, a target velocity and/or a target angular velocity of a control point of the mobile platform;
generating, based on the target velocity and/or the target angular velocity of the control point of the mobile platform, motion control instructions for the mobile platform; and
controlling, based on the motion control instructions for the mobile platform, the mobile platform to move.

9. The control method according to claim 8, wherein the method comprises:

determining, based on force vector information and/or torque vector information of the force sensor, a target velocity and/or a target angular velocity of the control point in a control point coordinate system; and

determining, based on a target velocity and/or a target angular velocity of the control point in a control point coordinate system and a transformation relationship between the control point coordinate system and the reference coordinate system, a target velocity and/or a target angular velocity of the control point relative to a reference coordinate system.

10. The control method according to claim 9, wherein determining a target velocity and/or a angular velocity of the control point in a control point coordinate system comprises:

determining, based on the force vector information and the transformation relationship between a force sensor coordinate system and the control point coordinate system, force vector information in a control point coordinate system; and

determining, based on a force proportional coefficient, a target velocity of the control point in a control point coordinate system; and/or

determining, based on the torque vector information and the transformation relationship between a force sensor coordinate system and the control point coordinate system, torque vector information in a control point coordinate system; and

determining, based on a torque proportional coefficient, a target angular velocity of the control point in a control point coordinate system.

11. The control method according to claim 9 or 10, wherein,

the control point coordinate system comprises a trocar coordinate system, a trocar fixing point coordinate system or remote center of motion (RCM) coordinate system; and/or

an origin of the reference coordinate system is located at an intersection of a ground and a proximal joint axis of the motion arm.

12. The control method according to any one of claims 8-11, wherein the motion arm, or the motion arm and the mobile platform, comprise one or more joints and one or more joint motors that drive the one or more joints, generating motion control instructions for the motion arm comprises:

determining, based on a target velocity and/or a target angular velocity of a control point of the motion arm relative to the reference coordinate system, joint values and joint velocities of the one or more joints; and

determining, based on joint values and joint velocities of the one or more joints, driving control instructions for the one or more joint motors.

13. The control method according to claim 12, wherein the one or more joints comprise:

a lifting joint of the mobile platform; and

a first transverse arm rotation joint, a second transverse arm rotation joint, a first arc arm rotation joint, a second arc arm rotation joint and a third arc arm rotation joint of the motion arm; or

the one or more joints comprise:

a first transverse arm rotation joint, a second transverse arm rotation joint, a lifting joint, a first arc arm rotation joint, a second arc arm rotation joint and a third arc arm rotation joint of the motion arm.

14. The control method according to any one of claims 8-13, wherein the method comprises:

determining, based on the force vector information and a transformation relationship between a force sensor coordinate system and the mobile platform control point coordinate system, force vector information in the mobile platform control point coordinate system; and/or

determining, based on force vector information in the mobile platform control point coordinate system, torque vector information in the mobile platform control point coordinate system.

15. The control method according to claim 14, wherein determining a target velocity and/or a target angular velocity of a control point of the mobile platform comprises:

determining, based on a forward direction component of force vector information in the mobile platform control point coordinate system, a force in a forward direction of the mobile platform; and

determining, based on a forward force proportional coefficient, a target forward velocity of the mobile platform; and/or

determining, based on a vertical direction component of torque vector information in the mobile platform control point coordinate system, a torque of turning of the mobile platform; and

determining, based on a turning torque proportional coefficient, a target angular velocity of the mobile platform; and/or

determining, based on a vertical direction component of force vector information in the mobile platform control point coordinate system, a force in a lifting direction of the mobile platform; and

determining, based on a lifting proportional coefficient, a target lifting velocity of the mobile platform.

16. The control method according to claim 15, wherein the mobile platform comprises one or more active casters and one or more caster motors driving the active casters, the method comprises:

determining, based on a target forward velocity of the mobile platform and a target angular velocity of the mobile platform, target velocities of the one or more active casters; and

determining, based on target velocities and radii of the one or more active casters, driving control instructions for the one or more active caster motors; and/or

the mobile platform comprises a column, a lifting joint connected with the column and a column lifting motor driving the column lifting joint, the method comprises:

determining, based on a target lifting velocity of the mobile platform, driving control instructions for the column lifting motor.

17. The control method according to claim 16, wherein,

the active casters comprise a first caster and a second caster that are disposed symmetrically;

a vertical height of an origin of the mobile platform control point coordinate system is the same as a vertical height of the reference coordinate system, a horizontal position of an origin of the mobile platform control point coordinate system is projected within the ground as a projection of a midpoint of the first caster and the second caster on the ground.

18. A surgical robot comprising:

a mobile platform;

a motion arm, the motion arm is movably disposed on the mobile platform;

a grip disposed on the motion arm and used to control motions of the motion arm and the mobile platform, the grip comprises at least one force sensor, the force sensor is used to detect a force or torque externally applied to the grip; and

a controller configured to execute the control method according to any one of claims 1-17.

19. A computer storage medium, wherein the storage medium comprises at least one instruction, the at least one instruction is executed by a processor to configure the processor to execute the control method according to any one of claims 1-17.

20. A computer system comprises:

a storage medium comprising at least one instruction; and

a processor configured to execute the at least one instruction to configure the processor to execute the control method according to any one of claims 1-17.

**1000**

Receiving input information corresponding to user
input from the input device
— 1001

Controlling the motion arm or the mobile station to
move based on the input information
— 1003

FIG. 1

100

Robot System

Mobile Station — 110

Motion Arm — 120

Input Device — 150

Controller — 140

FIG. 2

FIG. 3

FIG. 4

FIG. 5

## 2000

| Receiving force sensor information corresponding to the force and/or the torque applied to the grip from at least one force sensor | 2001 |

| Controlling the motion arm or the mobile station to move based on the force sensor information | 2003 |

FIG. 6

100

120    130    131

Robot System

110    Mobile Station    Motion Arm    Grip    Force Sensor

Controller

140

FIG. 7

3000

Determining the target velocity and/or the target angular velocity of the control point of the motion arm relative to the reference coordinate system based on the force sensor information    3001

Generating a motion control instruction for the motion arm based on the target velocity and/or the target angular velocity of the control point of the motion arm relative to the reference coordinate system    3003

Controlling the motion arm to move based on the motion control instruction for the motion arm    3005

FIG. 8

4000

| | |
|---|---|
| Determining the target velocity and/or the target angular velocity of the control point of the mobile station based on the force sensor information | 4001 |
| Generating a motion control instruction for the mobile station based on the target velocity and/or the target angular velocity of the control point of the mobile station | 4003 |
| Controlling the mobile station to move based on the motion control instruction for the mobile station | 4005 |

FIG. 9

FIG. 10

FIG. 11

FIG. 12

## INTERNATIONAL SEARCH REPORT

| | International application No. |
|---|---|
| | **PCT/CN2024/096927** |

| | |
|---|---|
| **A. CLASSIFICATION OF SUBJECT MATTER** | |
| A61B34/30(2016.01)i | |
| According to International Patent Classification (IPC) or to both national classification and IPC | |

| | |
|---|---|
| **B. FIELDS SEARCHED** | |

Minimum documentation searched (classification system followed by classification symbols)

IPC: A61B34/-

Documentation searched other than minimum documentation to the extent that such documents are included in the fields searched

Electronic data base consulted during the international search (name of data base and, where practicable, search terms used)

CNTXT, ENTXTC, DWPI: 手术机器人, 输入, 把手, 手柄, 操作器, 操作部, 触摸屏, 触控屏, 移动站, 台车, 推车, 塔, 移动, 运动, 定位, 臂, 力传感, 角速度, 模式; surgical robot, workstation, station, cart, arm, position???, orientat+, mov?????, motion

### C. DOCUMENTS CONSIDERED TO BE RELEVANT

| Category* | Citation of document, with indication, where appropriate, of the relevant passages | Relevant to claim No. |
|---|---|---|
| X | CN 114074328 A (BEIJING SURGERII TECHNOLOGY CO., LTD.) 22 February 2022 (2022-02-22)<br>  description, paragraphs [0016]-[0072], and figures 1-5(b) | 1-2, 6-7, 19-20 |
| Y | CN 114074328 A (BEIJING SURGERII TECHNOLOGY CO., LTD.) 22 February 2022 (2022-02-22)<br>  description, paragraphs [0016]-[0072], and figures 1-5(b) | 3-5, 8-18 |
| Y | CN 101999938 A (SHANGHAI JIAO TONG UNIVERSITY) 06 April 2011 (2011-04-06)<br>  description, paragraphs [0021]-[0033], and figures 1-4 | 3-5, 8-18 |
| Y | CN 114469351 A (INTUITIVE SURGICAL OPERATIONS, INC.) 13 May 2022 (2022-05-13)<br>  description, paragraphs [0171]-[0270] | 8-17 |
| X | CN 115568948 A (WUHAN UNITED IMAGING ZHIRONG MEDICAL TECHNOLOGY CO., LTD.) 06 January 2023 (2023-01-06)<br>  description, paragraphs [0054]-[0138], and figures 1-13 | 1-2, 4, 6-7, 19-20 |
| A | CN 111328306 A (AURIS HEALTH, INC.) 23 June 2020 (2020-06-23)<br>  entire document | 1-20 |

| ☑ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **11 September 2024** | **20 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/096927** |

| C. | DOCUMENTS CONSIDERED TO BE RELEVANT | | |
|---|---|---|
| Category* | Citation of document, with indication, where appropriate, of the relevant passages | Relevant to claim No. |
| A | CN 115444555 A (BEIJING CHANGMUGU MEDICAL TECHNOLOGY CO., LTD. et al.) 09 December 2022 (2022-12-09) entire document | 1-20 |
| A | US 2015066051 A1 (SAMSUNG ELECTRONICS CO., LTD.) 05 March 2015 (2015-03-05) entire document | 1-20 |

Form PCT/ISA/210 (second sheet) (July 2022)

INTERNATIONAL SEARCH REPORT
Information on patent family members

International application No.

**PCT/CN2024/096927**

| Patent document cited in search report | | | Publication date (day/month/year) | Patent family member(s) | | | Publication date (day/month/year) |
|---|---|---|---|---|---|---|---|
| CN | 114074328 | A | 22 February 2022 | EP | 4201606 | A1 | 28 June 2023 |
| | | | | US | 2023256607 | A1 | 17 August 2023 |
| | | | | WO | 2022037209 | A1 | 24 February 2022 |
| CN | 101999938 | A | 06 April 2011 | None | | | |
| CN | 114469351 | A | 13 May 2022 | US | 2017215976 | A1 | 03 August 2017 |
| | | | | US | 10123844 | B2 | 13 November 2018 |
| | | | | KR | 20130010093 | A | 25 January 2013 |
| | | | | KR | 101304272 | B1 | 11 September 2013 |
| | | | | EP | 2332477 | A2 | 15 June 2011 |
| | | | | EP | 2332477 | B1 | 07 March 2018 |
| | | | | KR | 20130010091 | A | 25 January 2013 |
| | | | | KR | 101304277 | B1 | 11 September 2013 |
| | | | | US | 2011276059 | A1 | 10 November 2011 |
| | | | | US | 8624537 | B2 | 07 January 2014 |
| | | | | US | 2011264109 | A1 | 27 October 2011 |
| | | | | US | 8749189 | B2 | 10 June 2014 |
| | | | | US | 2011264110 | A1 | 27 October 2011 |
| | | | | US | 8816628 | B2 | 26 August 2014 |
| | | | | US | 2011264108 | A1 | 27 October 2011 |
| | | | | US | 8786241 | B2 | 22 July 2014 |
| | | | | EP | 3973910 | A1 | 30 March 2022 |
| | | | | EP | 2332479 | A2 | 15 June 2011 |
| | | | | EP | 2332479 | B1 | 05 July 2017 |
| | | | | ATE | 544414 | T1 | 15 February 2012 |
| | | | | EP | 2332482 | A2 | 15 June 2011 |
| | | | | EP | 2332482 | B1 | 12 April 2017 |
| | | | | US | 2017215975 | A1 | 03 August 2017 |
| | | | | US | 10194998 | B2 | 05 February 2019 |
| | | | | US | 2021346107 | A1 | 11 November 2021 |
| | | | | US | 12029513 | B2 | 09 July 2024 |
| | | | | US | 2018042687 | A1 | 15 February 2018 |
| | | | | US | 10512514 | B2 | 24 December 2019 |
| | | | | US | 2011264112 | A1 | 27 October 2011 |
| | | | | US | 8749190 | B2 | 10 June 2014 |
| | | | | US | 2011270271 | A1 | 03 November 2011 |
| | | | | US | 8823308 | B2 | 02 September 2014 |
| | | | | US | 2017215974 | A1 | 03 August 2017 |
| | | | | US | 10117714 | B2 | 06 November 2018 |
| | | | | WO | 2006124390 | A2 | 23 November 2006 |
| | | | | US | 2020060777 | A1 | 27 February 2020 |
| | | | | US | 11534251 | B2 | 27 December 2022 |
| | | | | KR | 20130010092 | A | 25 January 2013 |
| | | | | KR | 101304278 | B1 | 11 September 2013 |
| | | | | EP | 2332478 | A2 | 15 June 2011 |
| | | | | EP | 2332478 | B1 | 16 August 2017 |
| | | | | US | 2018028268 | A1 | 01 February 2018 |
| | | | | US | 10512513 | B2 | 24 December 2019 |
| | | | | EP | 2332480 | A2 | 15 June 2011 |
| | | | | EP | 2332480 | B1 | 26 April 2017 |
| | | | | US | 2007013336 | A1 | 18 January 2007 |

Form PCT/ISA/210 (patent family annex) (July 2022)

## INTERNATIONAL SEARCH REPORT
### Information on patent family members

| | International application No. |
|---|---|
| | **PCT/CN2024/096927** |

| Patent document cited in search report | | | Publication date (day/month/year) | Patent family member(s) | | | Publication date (day/month/year) |
|---|---|---|---|---|---|---|---|
| | | | | US | 8004229 | B2 | 23 August 2011 |
| | | | | EP | 2332481 | A2 | 15 June 2011 |
| | | | | EP | 2332481 | B1 | 05 July 2017 |
| | | | | KR | 20080047318 | A | 28 May 2008 |
| | | | | KR | 101283407 | B1 | 08 July 2013 |
| | | | | US | 2015032126 | A1 | 29 January 2015 |
| | | | | US | 9687310 | B2 | 27 June 2017 |
| | | | | EP | 3231386 | A1 | 18 October 2017 |
| | | | | EP | 3231386 | B1 | 17 March 2021 |
| | | | | EP | 2332483 | A2 | 15 June 2011 |
| | | | | EP | 2332483 | B1 | 12 April 2017 |
| | | | | EP | 3231387 | A1 | 18 October 2017 |
| | | | | EP | 3231387 | B1 | 06 November 2019 |
| | | | | EP | 2332484 | A2 | 15 June 2011 |
| | | | | EP | 2332484 | B1 | 25 January 2017 |
| | | | | EP | 2332484 | B2 | 08 June 2022 |
| | | | | ES | 2381975 | T3 | 04 June 2012 |
| | | | | ES | 2381975 | T5 | 27 December 2022 |
| | | | | EP | 1885273 | A2 | 13 February 2008 |
| | | | | EP | 1885273 | B1 | 08 February 2012 |
| | | | | EP | 1885273 | B2 | 08 June 2022 |
| | | | | EP | 3231388 | A1 | 18 October 2017 |
| | | | | EP | 3231388 | B1 | 03 November 2021 |
| | | | | US | 2011264111 | A1 | 27 October 2011 |
| | | | | US | 8541970 | B2 | 24 September 2013 |
| | | | | US | 2015051733 | A1 | 19 February 2015 |
| | | | | US | 9554859 | B2 | 31 January 2017 |
| CN | 115568948 | A | 06 January 2023 | None | | | |
| CN | 111328306 | A | 23 June 2020 | AU | 2018347473 | A1 | 23 April 2020 |
| | | | | AU | 2018347473 | B2 | 12 January 2023 |
| | | | | US | 2023302658 | A1 | 28 September 2023 |
| | | | | US | 2019105785 | A1 | 11 April 2019 |
| | | | | US | 10434660 | B2 | 08 October 2019 |
| | | | | JP | 2020536755 | A | 17 December 2020 |
| | | | | JP | 7301819 | B2 | 03 July 2023 |
| | | | | US | 10016900 | B1 | 10 July 2018 |
| | | | | US | 2020039086 | A1 | 06 February 2020 |
| | | | | US | 11701783 | B2 | 18 July 2023 |
| | | | | KR | 20200071745 | A | 19 June 2020 |
| | | | | KR | 102587262 | B1 | 11 October 2023 |
| | | | | WO | 2019074670 | A1 | 18 April 2019 |
| | | | | EP | 3676060 | A1 | 08 July 2020 |
| | | | | EP | 3676060 | B1 | 06 March 2024 |
| CN | 115444555 | A | 09 December 2022 | None | | | |
| US | 2015066051 | A1 | 05 March 2015 | US | 9770300 | B2 | 26 September 2017 |
| | | | | EP | 2845556 | A1 | 11 March 2015 |
| | | | | EP | 2845556 | B1 | 17 August 2022 |
| | | | | KR | 20150027618 | A | 12 March 2015 |
| | | | | KR | 102306959 | B1 | 01 October 2021 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023109398598

- CN 2023108315579